# EUROPEAN PATENT APPLICATION

(11) **EP 2 048 136 A1**
(43) Date of publication of application: **15.04.2009**
(21) Application number: 07788680.2
(22) Date of filing: 17.07.2007
(51) Int. Cl.: C07D 265/36, A01N 43/84

(54) **PHYTOTOXIC HALOGENATED DERIVATIVES OF BENZOXAZINONES**

(30) Priority: 28.07.2006 ES 200602033
(71) Applicant: Universidad de Cadiz, 11001 Cadiz (ES)
(72) Inventor: MACÍAS DOMÍNGUEZ, Francisco Antonio, E-11510 Puerto Real (Cádiz) (ES); GONZÁLEZ MOLINILLO, José Maria, E-11510 Puerto Real (Cádiz) (ES); VARELA MONTOLLA, Rosa Maria, E-11510 Puerto Real (Cádiz) (ES); CHINCHILLA SALCEDO, Nuria, E-11510 Puerto Real (Cádiz) (ES); De SIQUEIRA, Joao Maximo, E-11510 Puerto Real (Cádiz) (ES); MARÍN MATEOS, David, E-11510 Puerto Real (Cádiz) (ES)
(74) Representative: ABG Patentes, S.L.
(86) International application number: PCT/ES2007/000438
(87) International publication number: WO 2008/012385

(57) **Abstract**

The invention relates to phytotoxic halogenated derivatives of benzoxazinones. The aim of the invention relates to the production of novel phytosanitary products that are suitable for agricultural weed control, based on natural chemical substances. The invention comprises novel chemical compounds having high levels of phytotoxic activity and the preparation methods thereof. Said substances are derived from natural products with the 1,4-benzoxazin-3-one skeleton involved in different chemical defense interactions in plants. The inventive compounds have suitable phytotoxic activity levels for use in weed control and for use as stable models for the development of novel phytosanitary products based on the 1,4-benzoxazin-3-one skeleton.

## Description

### Field of the Art

The invention belongs to the field of chemical compounds applicable to agricultural pest control.

### Prior State of the Art

The existence of a great variety of agricultural pests (weeds, parasitic plants, insects, fungi, bacteria) affects the yield and quality of agricultural crops.

A large amount and variet y of chemical pesticides is currently available the use of which raises problems related to the lack of selectivity on the different pests, the appearance of resistant pest varieties and environmental pollution.

Allelochemical agents and their synthetic analogues are an interesting alternative to traditional chemical pesticides, since they have novel mechanisms of action, they have a more specific interaction with the pests and cause less environmental damage. From this point of view, allelochemical agents and their analogues with phytotoxic activity have the qualities necessary for being adapted to the new pest control requirements, related to the development of sustainable agriculture models (see, for example: Duke, S.O.; Romagni, J.G.; Dayan, F.E. Crop Protection 2000, 19, 583-589).

For this reason, it is very interesting to obtain novel chemical substances intended for rational agricultural pest control.

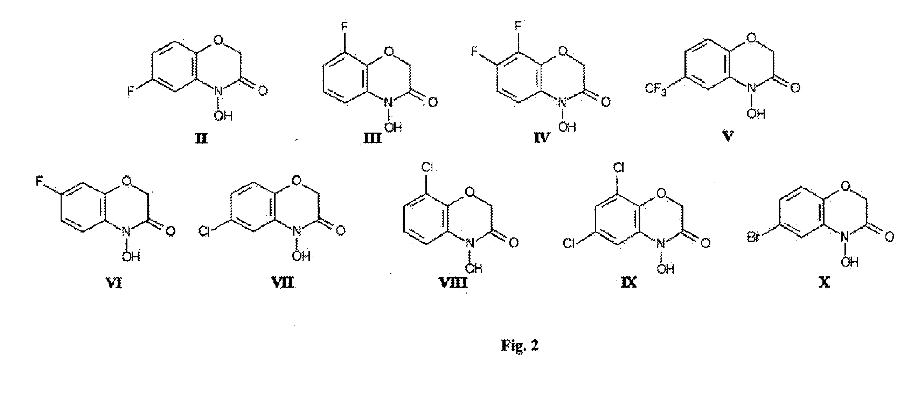

There is no precedent in relation to the synthesis or to the evaluation of the biological activity of the compounds 6-fluoro-4-hydroxy-(2*H*)-1,4-benzoxazin-3(4*H*)-one (I), 8-fluoro-4-hydroxy-(2H)-1,4-benzoxazin-3(4H)-one (II), 7,8-difluoro-4-hydroxy-(2*H*)-1,4-benzoxazin-3(4*H*)-one (III) and 6-trifluoromethyl-4-hydroxy-(2*H*)-1,4-benzoxazin-3(4*H*)-one (IV) (Figure 2). The compound 7-fluoro-4-hydroxy-(2H)-1,4-benzoxazin-3(4H)-one (V) (Figure 2) has been previously synthesized (see, for example, Geng, X.; Li, K.; Xu, F. Applied Microbiology and Biotechnology 2004, 64, 493-496) and its antimicrobial activity *(C. albicans,* E. *coli, S. aureus)* and anti-algal activity (C. *Xantella)* evaluated (see Bravo, H.R.; Lazo, W. J. Agric. Food Chem. 1996, 44, 1569-1571 and Bravo, H.R.; Lazo, W. Phytochemistry 1993, 33, 569-71). The activity values obtained for these compounds were moderate or low depending on the organism assayed.

The compound 6-chloro-(2*H*)-1,4-benzoxazin-3(4*H*)-one (VI) was synthetically obtained, without any of its biological activities being described (see Hashimoto, Y.; Ishizaki, T.; Shudo, K.; Okamoto, T. Chem. Pharm. Bull. 1983, 31, 3891-3896). Its isomer 8-chloro-(2*H*)-1,4-benzoxazin-3(4*H*)-one (VII) has been previously obtained in a synthetic manner, and evaluated by means of antifungal activity bioassays for pharmaceutical (Patent No. JP63185970) and antimicrobial use (see, for example Ozden, S.; Ozturk, A.M.; Goker, H.; Altanlar, N. Il Farmaco 2000, 55, 715-718). The derivative 6,8-dichloro-(2H)-1,4-benzoxazin-3(4*H*)-one (VIII) has been synthesized in previous studies on the chemistry of plant regulators (see Cavill, G.W.K.; Ford, D.L. J. Chem. Soc. Abstracts 1954, 565-568) and has been described as a fungicide (see patent No. DE1161080). The compound IX appears in a patent as an intermediate of the synthesis of compounds of pharmacological interest, active as PI3K inhibitors (see patent No. W02004052373), not forming part of any of the claims thereof. None of these compounds has been proposed as candidate for the development of novel molecules applicable to agricultural weed control.

### Description of the Invention

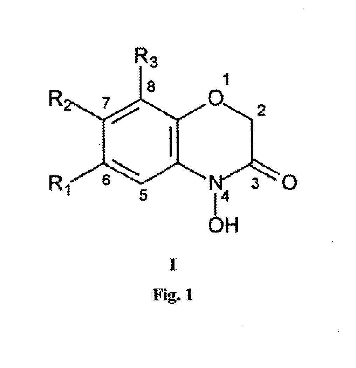

The invention comprises the compounds of general formula I (Figure 1) the molecular structure of which is defined as a 4-hydroxy-(2*H*)-1,4-benzoxazin-3(4*H*)-one base skeleton, wherein R₁ is a fluorine atom or a trifluoromethyl group if R₂ and R₃ are hydrogen atoms; R₁ is a chlorine atom if R₂ is a hydrogen atom and R₃ is a hydrogen or chlorine atom; R₁ is a bromine atom if R₂ and R₃ are hydrogen atoms; R₂ is a fluorine atom if R₁ is a hydrogen atom and R₃ is a hydrogen or fluorine atom; or R₃ is a fluorine or chlorine atom if R₁ and R₂ are hydrogen atoms. The chemical compounds object of the invention are those that satisfy the general formula I in the terms in which it is described: 6-fluoro-4-hydroxy-(2*H*)-1,4-benzoxazin-3(4*H*)-one (II), 8-fluoro-4-hydroxy-(2*H*)-1,4-benzoxazin-3(4*H*)-one (III), 7,8-difluoro-4-hydroxy-(2*H*)-1,4-benzoxazin-3(4*H*)-one (IV), 6-trifluoromethyl-4-hydroxy-(2*H*)-1,4-benzoxazin-3(4*H*)-one (V), 7-fluoro-4-hydroxy- (2*H*)-1,4-benzoxazin-3 (4*H*)-one (VI), 6-chloro-4-hydroxy-(2H)-1,4-benzoxazin-3(4H)-one (VII), 8-chloro-4-hydroxy-(2*H*)-1,4-benzoxazin-3(4*H*)-one (VIII), 6, 8-dichloro-4-hydroxy-(2*H*)-1,4-benzoxazin-3(4*H*)-one (IX) and 6-bromo-(2*H*)-1,4-benzoxazin-3(4*H*)-one (X) (Figure 2).

These compounds are models for the development of novel phytosanitary products based on the 1,4-benzoxazin-3-one skeleton involved in different ecological chemical defense interactions in plants (see, for example: Niemeyer, H.; Phytochemistry 1988, 11, 3349-3358) and having as an essential characteristic the presence of one or several fluorine or chlorine atoms in the aromatic ring or in the substituents thereof, the absence of oxygenated functional groups in position C-2, and the presence of a hydroxyl group bound to the nitrogen atom of position 4 (Figure 1).

### Brief Description of the Drawings

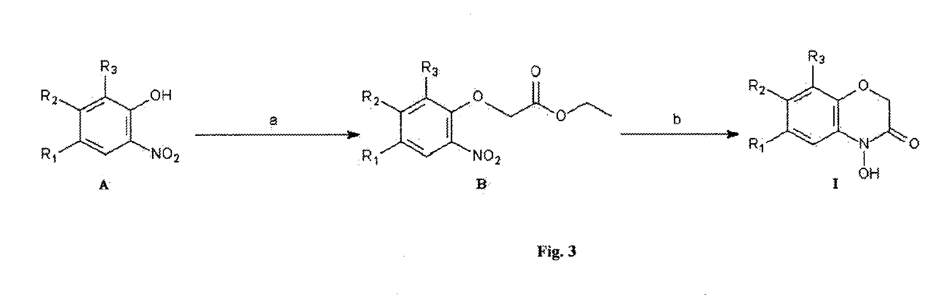

Several figures describing the compounds to which reference is made and the chemical transformations in which they are involved are attached to the text of the present specification.
Figure 1 shows the general formula of the compounds which are claimed.
Figure 2 shows the specific formulas of the compounds which are claimed.
   II.- 6-fluoro-4-hydroxy-(2*H*)-1,4-benzoxazin-3(4*H*)-one
   III.- 8-fluoro-4-hydroxy-(2*H*)-1,4-benzoxazin-3(4*H*)-one
   IV.- 7,8-difluoro-4-hydroxy-(2*H*)-1,4-benzoxazin-3(4*H*)-one
   V.- 6-trifluoromethyl-4-hydroxy-(2*H*)-1,4-benzoxazin-3(4*H*)-one
   VI.- 7-fluoro-4-hydroxy-(2*H*)-1,4-benzoxazin-3(4*H*)-one
   VII.- 6-chloro-4-hydroxy-(2*H*)-1,4-benzoxazin-3(4*H*)-one
   VIII- 8-chloro-4-hydroxy-(2*H*)-1,4-benzoxazin-3(4*H*)-one
   IX.- 6,8-dichloro-4-hydroxy-(2*H*)-1,4-benzoxazin-3(4*H*)-one
   X.- 6-bromo-4-hydroxy-(2*H*)-1,4-benzoxazin-3(4*H*)-one
Figure 3 shows the scheme of the synthesis method for compounds II to X. a) THF, KOH/EtOH, BrCH₂COOEt₅ 25°C, 12 h. b) H₂O/dioxane 1:1, NaBH₄, Pd/C.

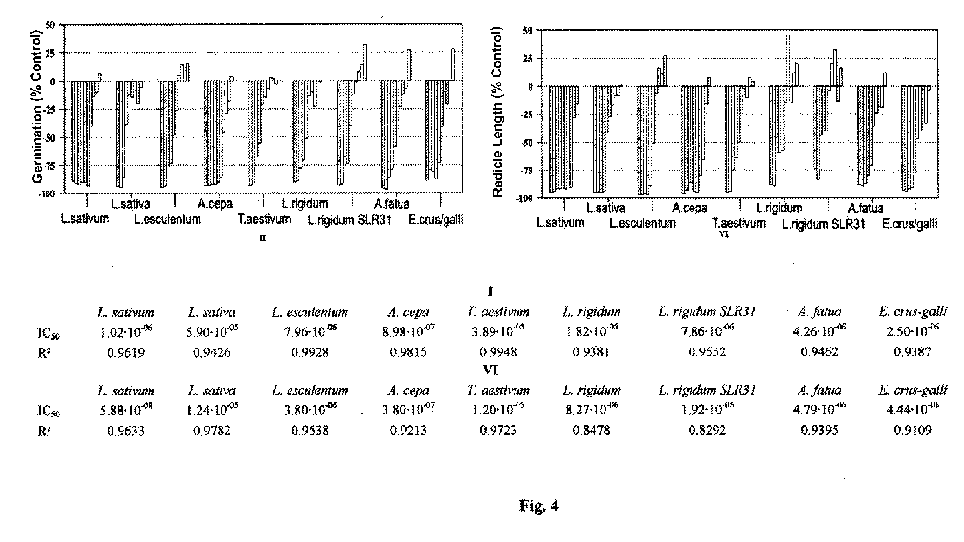

Figure 4 shows the phytotoxic activity of compounds II and VII. The biological activity data provided are presented in bar graph form, in which the zero value represents the control, positive values represent stimulation and negative values represent inhibition of the corresponding parameters measured. Accompanying said graph there is a table showing the values of IC₅₀ and their corresponding coefficients of determination obtained by means of fitting the activity data to a sigmoid dose-response relationship model with a constant slope. This data represents the concentration of the compounds which causes 50% of the phytotoxic effect observed, forming a parameter commonly used in the discussion of the biological activities of chemical products in general.

### Embodiment of the Invention. Examples of Preparation and Activity.

General method for the preparation of the compounds object of the invention: The compounds object of the invention are prepared in two steps (Figure 3). The first of them consists of a nucleophilic substitution reaction on a 2-nitrophenol with the suitable substitution pattern in the aromatic ring (A) with ethyl bromoacetate in basic medium, giving rise to an open-chain precursor compound (B). This compound is transformed into the corresponding product object of the invention (general formula I, Figure 1) by means of a reductive cyclization reaction with sodium borohydride in the presence of 10% activated carbon-supported palladium, in a 50% solution of 1,4-dioxane in water.

The generic protocols of each of the steps are the following:

General method for the preparation of compounds with type B structure (Figure 3): The compound with type A structure is dissolved in dry tetrahydrofuran (50 mg/mL) in an inert atmosphere. One equivalent of 0.1 N potassium hydroxide in absolute ethanol is then added. The corresponding potassium alkoxide is obtained after 30 minutes of reaction and by distillation under reduced pressure, to which dry N,N-dimethylformamide is added until its complete dissolution. Subsequently, 1.2 equivalents of ethyl bromoacetate are added in an inert atmosphere. After 16 hours of reaction, an equivalent volume of ethyl acetate is added to the solution and N,N-dimethylformamide is removed by extracting with 5 portions of distilled water of the same volume as the ethyl acetate added. The resulting organic phase is treated with anhydrous sodium sulfate, filtered and distilled under reduced pressure to obtain the compound of structural type B.

Preparation of compounds of general formula I (Figure 3): A volume of 12 mL of a 50% solution of 1,4-dioxane in water is prepared, to which 10% palladium on carbon (2.5 mg/mL) and sodium borohydride (25 mg/mL) is added with stirring. The compound of structural type B (20.25 mg/mL) is then gradually added to the solution, from a solution prepared with the minimal amount necessary of pure 1,4-dioxane. After two hours of reaction, the catalyst is removed by vacuum filtration and a 0.1 N solution of hydrochloric acid is added to the filtrate until reaching a value of pH=4. The solution thus obtained is taken to a separating funnel and extracted with 3 portions of an equivalent volume of ethyl acetate. The resulting organic phases are pooled and treated with anhydrous sodium sulfate. After filtration and evaporation under reduced pressure, the resulting crude reaction product is purified by silica gel column chromatography with elution with a solution of hexane and 40% ethyl acetate to give the compound of structural type I.

### Example 1

### Obtaining compound II (Figure 3)

### • Preparation of the corresponding compound of structural type B (Figure 3)

500 mg of the compound of structural type A corresponding to compound II (4-fluoro-2-nitrophenol) are dissolved in 10 mL of dry tetrahydrofuran in an inert atmosphere. Then, 31.8 mL of a 0.1 N solution of potassium hydroxide in absolute ethanol are added. After 30 minutes of reaction and by distillation under reduced pressure the corresponding potassium alkoxide is obtained, which is dissolved in 100 mL of dry N,N-dimethylformamide. 422 µL of ethyl bromoacetate are added in an inert atmosphere. After 16 hours of reaction, 100 mL of ethyl acetate are added to the solution and N,N-dimethylformamide is removed by extraction with 5 portions of 100 mL of distilled water. The resulting organic phase is treated with anhydrous sodium sulfate, filtered and distilled under reduced pressure to obtain the compound of structural type B corresponding to compound II (ethyl 4-fluoro-2-nitrophenoxyacetate).

### • Preparation of compound II

12 mL of a 50% solution of 1,4-dioxane in water are prepared, to which 30 mg of 10% palladium on carbon and 74 mg of sodium borohydride are added with stirring. Then, 243 mg of the compound obtained in the previous step are dissolved in 1,4-dioxane (5 mL) and gradually added to the previously prepared suspension. After two hours of reaction, the catalyst is removed by vacuum filtration and a 0.1 N solution of hydrochloric acid is added to the filtrate until reaching a value of pH=4. The solution thus obtained is taken to a separating funnel and extracted with 3 portions of 50 mL of ethyl acetate. The resulting organic phases are pooled and treated with anhydrous sodium sulfate. After filtration and evaporation under reduced pressure, the resulting crude reaction product is purified by silica gel column chromatography with elution with a solution of hexane and 40% ethyl acetate to give compound II.

The spectroscopic data obtained for the compounds object of the invention, all prepared in a manner similar to that described above, are the following:
6-Fluoro-(2*H*)-1,4-benzoxazin-3(4*H*)-one (II): ¹H NMR (DMSO-d₆, 400 MHz): δ 4.73 (2H, s, CH2-2), 6.80 (1H, dd, 2 and 8 Hz, H-5); 6.98 (2H, m, H-7, H-8). ¹³C NMR (DMSO-d₆, 75 MHz): 67.9 (C-2); 100.6 (C-5); 109.4 (C-7); 117.1 (C-8); 139.8 (C-10); 156.1 (C-9); 159.2 (C-6); 160.7 (C-3). EI-MS m/z (%): 199.1 (80%) [M]⁺
8-Fluoro-(2*H*)-1,4-benzoxazin-3(4*H*)-one (III): ¹H NMR (MeOH-d₄, 400 MHz): δ 4.80 (2H, s, CH2-2), 6.88 (1H, ddd, 10, 8, and 2 Hz, H-7); 7.02 (1H, ddd, 5, 8, and 9 Hz, H-6); 7.11 (1H, ddd, 2, 3 and 9 Hz). ¹³C NMR (MeOH-d₄, 75 MHz): 69.3 (C-2); 109.8 (C-5); 112.4 (C-7); 123.5 (C-6); 133.4 (C-9); 139.0 (C-10); 151.0 (C-9), 152.0 (C-8), 162.1 (C-3). EI-MS m/z (%): 199.1 (80%) [M]⁺
7,8-Difluoro-(2*H*)-1,4-benzoxazin-3(4*H*)-one (IV): ¹H NMR (MeOH-d₄, 400 MHz): δ 4.84 (2H, s, CH2-2), δ 6.96 (1H, ddd, 7.6, 9.2 and 10.0 Hz, H-6); δ 7.08 (1H, ddd, 2.0, 4.8 and 9.2 Hz, H-5). ¹³C NMR (MeOH-d₄, 75 MHz): 69.6 (C-2); 108.5 (C-5); 110.5 (br d, 19.0 Hz, C-6); 128.5 (C-4a); 135.3 (C-8a); 141.2 (dd, 246 and 16.0 Hz, C-8), 149.05 (dd, 241.0 and 10 Hz, C-7); 161.8 (C-3). EI-MS m/z (%): 201.1 [M]⁺
6-Trifluoromethyl-(2*H*)-1,4-benzoxazin-3(4*H*)-one (V): ¹H NMR (CDCl₃, 400 MHz): δ 4.82 (2H, s, CH₂-2), δ 7.03 (1H, dd, 8.4 and 0.4 Hz, H-8); δ 7.32 (1H, br t, 8.4 and 2.0 Hz, H-7), δ 7.64 (1H, br d, 2.0 Hz, H-5). ¹³C NMR (CDCl₃, 75 MHz): 67.6 (C-2); 111.3 (d, 3.75 Hz, C-8); 116.7 (d, 4.28 Hz, C-5), 122.6 (d, 3.8 Hz, C-7); 125.2 (q, 33 Hz, C-6); 123.7 (q, 270 Hz, CF₃); 127.9 (C-4a); 146.1 (C-8a); 160.1 (C-3). EI-MS m/z (%): 233.2 [M]⁺ (75%).
7-Fluoro-(2*H*)-1,4-benzoxazin-3(4*H*)-one (VI): ¹H NMR (MeOH-d₄, 400 MHz) : δ 4.74 (2H, s, CH2-2), 6.76 (2H, m, H-5, H-8) ; 7.29 (1H, dd, 5 and 8 Hz, H-6). ¹³C NMR (MeOH-d₄, 75 MHz) : 69.3 (C-2) ; 105.0 (C-8); 109.8 (C-6); 115.2 (C-10); 127.1 (C-10); 146.4 (C-9); 159.6 (C-3); 161.9 (C-7). EI-MS m/z (%): 199.1 (80%) [M]⁺
6-Chloro-(2*H*)-1,4-benzoxazin-3(4K)-one (VII): ¹H NMR (DMSO-d₆, 400 MHz): δ 4.77 (2H, s, CH2-2), 7.00 (1H, br d, 8.8 Hz, H-8); 7.03 (1H, br dd, 8.8 and 2.4 Hz, H-7) and 7.17 (1H, br d, 2.4 Hz, H-5). ¹³C NMR (DMSO-d₆, 75 MHz) : 67.8 (C-2) ; 112.6 (C-8); 117.6 (C-5); 123.2 (C-7); 126.4 (C-6); 131.0 (C-4a); 142.6 (C-8a); 160.3 (C-3). EI-MS m/z (%): 199.1 (80%) [M]⁺
8-Chloro- (2H) -1, 4-benzoxazin-3 (4H) -one (VIII): ¹H NMR (DMSO-d₆, 400 MHz): δ 4.87 (2H, s, CH2-2), δ 7.04 (1H, br t, J 8.4 Hz, H-6); δ 7.12 (1H, br dd, 8.0 and 1.2 Hz, H-5) and δ 7.18 (1H, dd, 8.0 and 1.2 Hz, H-7). ¹³C NMR (DMSO-d₆, 75 MHz) : δ 68.1 (C-2); δ 112.0 (C-5); δ 120.1 (C-8); δ 123.1 (C-6); δ 124.2 (C-7); δ 131.0 (C-4a); δ 139.7 (C-8a); δ 160.0 (C-3). EI-MS m/z (%): 199.0 [M]⁺ (45%).
6,8-Dichloro-(2*H*)-1,4-benzoxazin-3(4*H*)-one (IX): ¹H NMR (DMSO-d₆, 400 MHz): δ 4.91 (2H, s, CH2-2), δ 7.15 (1H, d, 1.8 Hz); δ 7.27 (1H, d, 1.8 Hz). ¹³C NMR (DMSO-d₆, 75 MHz): 68.0 (C-2); 111.6 (C-5); 120.9 (C-8); 123.1 (C-7); 126.3 (C-6); 131.8 (C-4a); 138.7 (C-8a); 160.0 (C-3) EI-MS m/z (%): 217.0 [M]⁺ (80%).
6-Bromo-(2*H*)-1,4-benzoxazin-3(4*H*)-one (X): NMR (MeOH-d₄, 400 MHz) : δ 4.55 (2H, s, CH2-2), 6.67 (1H, d, 8 Hz, H-8); 6.96 (1H, dd, 8 and 2 Hz, H-7); 7.31 (1H, d, 2 Hz, H-5). ¹³C NMR (MeOH-d₄, 75 MHz): 67.7 (C-2); 114.6 (C-6); 116.1 (C-8); 117.4 (C-5); 127.7 (C-7); 129.9 (C-10), 142.8 (C-9), 160.6 (C-3). EI-MS m/z (%): 243 [M(⁷⁹Br)]⁺ (98%), 245 [M, (⁸¹Br)]⁺ (1000).

### Example 2

### Biological activities of compounds II and VII (Figure 3)

The phytotoxicity bioassays for the compounds object of the invention are performed by means of the protocol of phytotoxicity bioassays in Petri dishes developed by the Grupo de Alelopatía de Cádiz (Cádiz Allelopathy Group) (see, for example: Macias, F.A.; Castellano, D.; Molinillo, J.M.G.; J. Agric. Food Chem. 2000, 48, 6, 2512-2521).

This assay attempts to imitate the natural conditions of the action of the products to be assayed on seeds of selected plant species, therefore the products are supplied in the form of aqueous solution in a Petri dish in which a certain number of seeds of each receiving species is introduced. The concentration range used allows observing the relationship between the phytotoxic activity of the product and its concentration. The dilutions assayed were 10⁻³ M, 5·10⁻⁴ M, 10⁻⁴ M, 5·10⁻⁵ M, 10⁻⁵ M, 5·10⁻⁶ M, 10⁻⁶ M, 5·10⁻⁷, 10⁻⁷ and 5·10⁻⁸. These solutions are buffered at pH=6 using to that end a 10 mM solution of 2-[N-morpholino]-ethanesulfonic acid (MES) the pH of which is adjusted to the desired value by means of adding a 1M solution of NaOH. The blank of this experiment is distilled and deionized water also buffered with MES but in which there is no product dissolved. The selection of the receiving plant species for the bioassay was performed based on a broad study carried out by this group, which is detailed in the previously mentioned publication, the chosen species being the monocotyledonous species *Allium cepa* L. (onion), Triticum aestivum L. (wheat) and the dicotyledonous species *Lepidium sativum* L. (watercress), *Lycopersicon esculentum* Will. (tomato), and *Lactuca sativa* L. (lettuce). To complete the phytotoxicity profile the species of monocotyledonous weeds *Avena fatua* L. (wild oat), *Echinochloa crus galli* L. P./Beauv. and *Lolium rigidum* L. (ryegrass) with two biotypes: the common biotype and the biotype multiresistant to herbicides SLR31, are added to the bioactivity assay. All the seeds are in the dark during the period in which the bioassay is performed and at a temperature of 25°C. The incubation time and the number of seeds and replicas to be assayed for each dilution have been optimized taking into account the characteristics of each seed.

The data acquisition and the statistical treatment of the results are performed using the computer support FITOMED^{®} (Automated system for the simultaneous acquisition and computerized management of variable-length measurements), patented by the University of Cádiz (P9901565). This system is formed by a digitizing table and an optical pencil (to acquire the lengths of radicles and hypocotyls) controlled by a personal computer provided with a software which collects data reducing the measurement acquisition time and performs the statistical treatment of such data and the graphic representation thereof.

All the compounds object of the invention had potent inhibitory activities on the germination and the development of model and weed species. The values of IC₅₀ for the radicle length, according to sigmoid type dose-response models, accompanied by the corresponding values of the coefficient of determination (R²) for the fits are shown in Table 1. The effects of the compounds assayed are extraordinarily potent, reaching values of IC₅₀ in the order of 58 nM (compound VII, L. sativum). Especially remarkable are the results on the weed species, in which the compounds were significantly active at concentrations less than one micromole per liter. Of the weed species assayed, the most affected were A. *fatua* and E. *crus galli.* In relation to the L. *rigidum* biotypes assayed, for the case of compound II, the multiresistant biotype was the most affected, whereas the opposite trend is observed for compound VII. The effects of both compounds on all the weeds were greater than against *Triticum aestivum* L., which is of great interest from the point of view of the selectivity of the compounds assayed and the consequences that this fact has on their applicability.

## Claims

1. Compounds of general formula I (Figure 1) the molecular structure of which is defined as a 4-hydroxy-(2*H*)-1,4-benzoxazin-3(4*H*)-one base skeleton, wherein R₁ is a fluorine atom or a trifluoromethyl group if R₂ and R₃ are hydrogen atoms; R₁ is a chlorine atom if R₂ is a hydrogen atom and R₃ is a hydrogen or chlorine atom; R₁ is a bromine atom if R₂ and R₃ are hydrogen atoms; R₂ is a fluorine atom if R₁ is a hydrogen atom and R₃ is a hydrogen or fluorine atom; or R₃ is a fluorine or chlorine atom if R₁ and R₂ are hydrogen atoms.

2. The compound 6-fluoro-4-hydroxy-(2*H*)-1,4-benzoxazin-3(4*H*)-one of general formula I according to claim 1, wherein R₁ is a fluorine atom and R₂ and R₃ are hydrogen atoms (II, Figure 2).

3. The compound 8-fluoro-4-hydroxy-(2*H*)-1,4-benzoxazin-3(4*H*)-one of general formula I according to claim 1, wherein R₁ and R₂ are hydrogen atoms and R₃ is a fluorine atom (III, Figure 2).

4. The compound 7,8-difluoro-4-hydroxy-(2H)-1,4-benzoxazin-3(4*H*)-one of general formula I according to claim 1, wherein R₁ is a hydrogen atom and R₂ and R₃ are fluorine atoms (IV, Figure 2).

5. The compound 6-trifluoromethyl-4-hydroxy-(2H)-1,4-benzoxazin-3(4*H*)-one of general formula I according to claim 1, wherein R₁ is a trifluoromethyl group and R₂ and R₃ are hydrogen atoms (V, Figure 2).

6. A preparation method for the compound 6-fluoro-4-hydroxy-(2*H*)-1,4-benzoxazin-3(4*H*)-one (II) of general formula I according to claims 1 and 2, **characterized by** a nucleophilic substitution reaction from 4-fluoro-2-nitrophenol with ethyl bromoacetate in basic medium, and a reductive cyclization reaction of the resulting product with sodium borohydride in the presence of activated carbon-supported palladium in a solution of water and 50% 1,4-dioxane.

7. A preparation method for the compound 8-fluoro-4-hydroxy-(2*H*)-1,4-benzoxazin-3(4*H*)-one (III) of general formula I according to claims 1 and 3, **characterized by** a nucleophilic substitution reaction from 2-fluoro-5-nitrophenol with ethyl bromoacetate in basic medium, and a reductive cyclization reaction of the resulting product with sodium borohydride in the presence of activated carbon-supported palladium in a solution of water and 50% 1,4-dioxane.

8. A preparation method for the compound 7,8-difluoro-4-hydroxy-(2*H*)-1,4-benzoxazin-3(4*H*)-one (IV) of general formula I according to claims 1 and 4, **characterized by** a nucleophilic substitution reaction from 2,3-difluoro-6-nitrophenol with ethyl bromoacetate in basic medium, and a reductive cyclization reaction of the resulting product with sodium borohydride in the presence of activated carbon-supported palladium in a solution of water and 50% 1,4-dioxane.

9. A preparation method for the compound 6-trifluoromethyl-4-hydroxy-(2*H*)-1,4-benzoxazin-3(4*H*)-one (V) of general formula I according to claims 1 and 5, **characterized by** a nucleophilic substitution reaction from 2-nitro-4-(trifluoromethyl)-phenol with ethyl bromoacetate in basic medium, and a reductive cyclization reaction of the resulting product with sodium borohydride in the presence of activated carbon-supported palladium in a solution of water and 50% 1,4-dioxane.

10. The use of the compounds 6-fluoro-4-hydroxy-(2H)-1,4-benzoxazin-3(4*H*)-one (II, Figure 2), 8-fluoro-4-hydroxy-(2*H*)-1,4-benzoxazin-3(4*H*)-one (III, Figure 2), 7,8-difluoro-4-hydroxy-(2*H*)-1,4-benzoxazin-3(4*H*)-one (IV, Figure 2), 6-trifluoromethyl-4-hydroxy-(2*H*)-1,4-benzoxazin-3(4*H*)-one (V, Figure 2), 7-fluoro-4-hydroxy-(2*H*)-1,4-benzoxazin-3(4*H*)-one (VI, Figure 2), 6-chloro-4-hydroxy-(2*H*)-1,4-benzoxazin-3(4*H*)-one (VII, Figure 2), 8-chloro-4-hydroxy-(2*H*)-1,4-benzoxazin-3(4*H*)-one (VIII, Figure 2), 6,8-dichloro-4-hydroxy-(2*H*)-1,4-benzoxazin-3(4*H*)-one (IX, Figure 2) and 6-bromo-4-hydroxy-(2*H*)-1,4-benzoxazin-3(4*H*)-one (X, Figure 2) as products having suitable phytotoxic activity levels for use in weed control and for use as stable models for the development of novel phytosanitary products based on the 1,4-benzoxazin-3-one skeleton.
